(19) Europäisches Patentamt
European Patent Office
Office européen des brevets

(11) EP 3 710 140 B1

(12) **EUROPÄISCHE PATENTSCHRIFT**

(45) Veröffentlichungstag und Bekanntmachung des Hinweises auf die Patenterteilung:
**25.03.2026 Patentblatt 2026/13**

(21) Anmeldenummer: **18811154.6**

(22) Anmeldetag: **12.11.2018**

(51) Internationale Patentklassifikation (IPC):
***B01D 61/12*** *(2006.01)* ***A61M 1/16*** *(2006.01)*
***B01D 61/58*** *(2006.01)* ***G16H 40/60*** *(2018.01)*
***B01D 61/02*** *(2006.01)* ***B01D 61/24*** *(2006.01)*

(52) Gemeinsame Patentklassifikation (CPC):
**B01D 61/025; A61M 1/1656; B01D 61/12; B01D 61/243; B01D 61/58; G16H 20/40; G16H 40/20; G16H 40/63;** B01D 2313/60; B01D 2313/70; B01D 2313/701; B01D 2313/903

(86) Internationale Anmeldenummer:
**PCT/EP2018/080875**

(87) Internationale Veröffentlichungsnummer:
**WO 2019/092232 (16.05.2019 Gazette 2019/20)**

(54) **ÜBERPRÜFUNG EINER UMKEHROSMOSE-ANLAGE ZUR ANWENDUNG BEI DIALYSEGERÄTEN**

EXAMINATION OF A REVERSE OSMOSIS INSTALLATION FOR USE WITH DIALYSIS DEVICES

VÉRIFICATION D’UNE INSTALLATION D’OSMOSE INVERSE POUR ÊTRE UTILISÉE DANS DES APPAREILS DE DIALYSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.11.2017 DE 102017126592**

(43) Veröffentlichungstag der Anmeldung:
**23.09.2020 Patentblatt 2020/39**

(73) Patentinhaber: **Fresenius Medical Care Deutschland GmbH**
**61352 Bad Homburg (DE)**

(72) Erfinder:
- **RUFF, Christian**
  **61440 Oberursel (DE)**
- **POHL, Thomas**
  **61381 Friedrichsdorf (DE)**
- **WILD, Michael**
  **87437 Kempten (DE)**

(74) Vertreter: **Schwarz, Claudia et al**
**Schwarz + Kollegen**
**Patentanwälte**
**Heilmannstraße 19**
**81479 München (DE)**

(56) Entgegenhaltungen:
**WO-A1-00/36412**
**WO-A1-2010/024963**
**CN-A- 106 110 889**
**FR-A1- 2 911 687**
**US-A1- 2002 077 777**
**US-A1- 2002 130 069**
**US-A1- 2004 138 840**
**US-A1- 2011 284 480**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 710 140 B1

**Beschreibung**

Die vorliegende Erfindung liegt auf dem Gebiet der Wassertechnologie im medizinischen Bereich und betrifft insbesondere die Sicherheitsüberprüfung von Umkehrosmose-Anlagen, die für medizintechnische Geräte, insbesondere Dialysegeräte, verwendet werden, um das benötigte Wasser in ausreichender Qualität und frei von (bakteriellen) Kontaminationen bereitstellen zu können. Die Erfindung bezieht sich insbesondere auf ein System, auf eine Analyseeinrichtung, eine Umkehrosmose-Anlage, einen Server und ein Verfahren zur Sicherheitsüberprüfung von zugeführtem Wasser und einem Computerprogrammprodukt.

Dialysegeräte werden mit Reinstwasser betrieben. Um das Reinstwasser bereitstellen zu können, werden Umkehrosmose-Anlagen (aufgrund der englischen Bezeichnung im Folgenden kurz: RO-Anlagen) eingesetzt.

Das grundlegende physikalische Prinzip der Umkehrosmose dient zur Konzentrierung von in Flüssigkeiten gelösten Stoffen. Dabei wird mit Druck der natürliche Osmose-Prozess umgekehrt. Schematisch lässt sich der RO-Prozess derart beschreiben, dass zwei Behältnisse mit Flüssigkeit (z.B.) Wasser von ungleichem Stoff- und insbesondere Salzgehalt gefüllt sind, die durch eine semipermeable Membran voneinander getrennt sind. Nach Anwendung eines osmotischen Drucks in dem Behältnis, in dem die Konzentration erhöht werden soll, können die Moleküle des Lösungsmittels gegen ihre "natürliche" osmotische Ausbreitungsrichtung wandern. Dabei muss der applizierte Druck höher sein als der Druck, der durch das osmotische Verlangen zum Konzentrationsausgleich entsteht. Das Verfahren drückt die Moleküle des Lösungsmittels in das Kompartiment, in dem gelöste Stoffe weniger konzentriert vorliegen. Mit diesem Verfahren verringert sich also die Konzentration von unerwünschten Stoffen auf der Reinwasserseite.

Das mittels der RO-Anlage hergestellte Wasser wird im medizinischen Bereich und insbesondere zum Betreiben von Dialysegeräten, wie beispielsweise dem Hämodialysesystem 5008 von Fresenius Medical Care und anderen extrakorporalen Blutbehandlungsgeräten, benötigt und dort zugeführt.

Für die Einhaltung von strengen Sicherheitsanforderungen der medizintechnischen Geräte ist es unabdingbar, dass das Wasser in der erforderlichen Qualität bereitgestellt wird. Dazu wird das von der RO-Anlage hergestellte Reinstwasser in definierten Zeitabständen auf die Einhaltung der chemischen und mikrobiologischen Sicherheitsanforderungen untersucht. Dies erfolgt in externen Laboren. Die Bedingungen zur Untersuchung sind in dem Standard ISO 23500 "Guidance for the preparation and quality management of fluids for haemodialysis and related therapies" definiert.

In der Regel wird der RO-Anlage bzw. der angeschlossenen Ringleitung eine Wasserprobe von Reinstwasser entnommen und einem Labor zugesendet. Dieses benötigt üblicherweise mehrere Tage zur Bereitstellung eines Laborberichts oder -Ergebnisses, das im Stand der Technik per Post oder Telefonanruf an den Betreiber der RO-Anlage übermittelt wird.

Das oben geschilderte Vorgehen nach dem Stand der Technik hat aber den durchaus nicht unerheblichen Nachteil, dass es mitunter mehrere Tage bis zu einer Woche dauern kann, bis das Laborergebnis auf der lokalen Anlage zur Verfügung steht. Falls das Ergebnis z.B. eine Kontamination mit bakteriellen Erregern oder einen anderen Sicherheitsdefekt indiziert, können die angeschlossenen Dialysegeräte erst von der RO-Anlage abgeschaltet werden, nachdem die Information lokal an die RO-Anlage übermittelt worden ist. In dieser Zeitphase besteht ein Sicherheitsrisiko, weil die Geräte weiterhin mit schlechter Wasserqualität betrieben werden. In einem solchen Fall stellt das bisherige Vorgehen somit eine Sicherheitslücke dar.

Die Verwendung von Sensoren zur Überprüfung von RO-Anlagen ist im Stand der Technik bekannt., So offenbart die WO 2010/024963 A1 inline Sensoren und die US 2011/0284480 A1 beschreibt Permeatsensoren für eine RO-Anlage.

Weiter sind im Stand der Technik Überwachungs- und/oder Warnsysteme bekannt, die die Funktion einer RO-Anlage vorwiegend im nicht-medizinischen Bereich prüfen, wie die CN 106119889A, die US2002/0077777 A1 und die US 2004/0138840 A1. Die FR 2 911 687 offenbart ein Überwachungssystem für Trinkwasser auf Einhaltung von regulatorischen Erfordernissen und beschreibt in einem Beispiel eine medizinische Anwendung unter Verwendung von Wasserproben.

Überwachungseinheiten von RO-Anlagen im Allgemeinen auf Basis von Sensordaten sind weiter aus der US 2002/0130069 A1 und der WO 00/36412 bekannt.

Ausgehend von dem bekannten Vorgehen nach dem Stand der Technik hat sich die vorliegende Erfindung deshalb zur Aufgabe gestellt, die Sicherheit von RO-Anlagen und daran angeschlossenen medizintechnischen Geräten zu verbessern. Zudem sollen die Analysefähigkeit der gesammelten erfassten Daten (Sensordaten, Laborwerte etc.) verbessert werden. Zudem sollen die sicherheitsrelevanten Daten früher bereitgestellt werden. Weiterhin soll die bereitgestellte Information mittels technischer Meldungen verbessert werden und unmittelbar lokal am Gerät verwertbar sein.

Diese Aufgabe wird erfindungsgemäß durch ein elektronisches Sicherheitssystem, eine RO-Anlage, einen Server und ein Verfahren zur Sicherheitsüberprüfung von zugeführtem Wasser und einem Computerprogrammprodukt gemäß den beiliegenden, einander nebengeordneten Patentansprüchen gelöst.

Im Folgenden wird die Erfindung anhand der systemgemäßen Aufgabenlösung und somit u.a. anhand eines Sicherheitssystems beschrieben. Dabei erwähnte Merkmale, Vorteile oder alternative Ausführungsformen sind ebenso auch auf die anderen beanspruchten Gegenstände zu übertragen und umgekehrt. Mit anderen Worten können auch die anderen gegenständlichen Ansprüche (die beispielsweise auf die Analyseeinrichtung, die RO-Anlage oder den Server gerichtet sind) und die Verfahrensansprüche mit den Merkmalen weitergebildet sein, die in Zusammenhang mit dem System beschrieben oder beansprucht sind. Die entsprechenden funktionalen Merkmale des Verfahrens werden dabei durch entsprechende gegenständliche Module, insbesondere durch elektronische Hardware-Module oder Mikroprozessor-Module, des Systems bzw. der Vorrichtung ausgebildet und umgekehrt.

Gemäß einem ersten Aspekt betrifft die Erfindung ein elektronisches Sicherheitssystem gemäß Anspruch 1 für eine RO-Anlage, wobei das Sicherheitssystem als zentrales, Server- und insbesondere Cloud-basiertes System zur Sicherstellung von ausreichender Reinstwasserqualität betrieben werden kann, und wobei die RO-Anlage für eine Anwendung und/oder für einen Betrieb mit einem Verbund von medizintechnischen Geräten, nämlich Dialysegeräten, ausgebildet ist.

Gemäß einem Aspekt der Erfindung kann das Sicherheitssystem umfassen:

- die RO-Anlage, die zur Herstellung von Reinstwasser bestimmt und die mit einer Sensoreinheit zur Erfassung von Sensordaten, insbesondere einer Leitfähigkeit vor und nach der Membran mit einem Rückhaltevermögen, ausgebildet ist und wobei die RO-Anlage eine elektronische Datenschnittstelle zum Austausch von analogen und/oder digitalen Daten umfasst, um die von der Sensoreinheit erfassten Sensordaten an eine externe Instanz außerhalb der RO-Anlage zu versenden;
- eine Analyseeinrichtung, die z.B. in einem Labor mit einer labortechnischen Vorrichtung zur Untersuchung bzw. Analyse der Wasserqualität einer Wasserprobe der RO-Anlage in Hinblick auf Sicherheitserfordernisse zu analysieren und wobei das Laborsystem zur Detektion von Kontaminationen des Wassers sowie zur Durchführung einer biologischen und/oder chemischen Analyse sowie von bakteriologischen Untersuchungen dient ausgebildet sein kann und wobei Analyseeinrichtung dazu bestimmt ist, Ergebnisdaten in Antwort auf die Analyse der Wasserprobe zu erzeugen, wobei die Analyseeinrichtung weiterhin mit einer Analyseschnittstelle ausgebildet ist, um die erzeugten Ergebnisdaten in elektronischer Form an eine externe Instanz außerhalb der Analyseeinrichtung zu versenden;
- ein Netzwerk zum Datenaustausch zwischen medizintechnischen Einrichtungen des Sicherheitssystems, nämlich zwischen der RO-Anlage und der Analyseeinrichtung und
- einen Server mit einer Auswerteeinrichtung, auf dem die erfassten und erzeugten Daten aggregiert werden und der zur konzertierten Verarbeitung der Daten dient, wobei die Ergebnisdaten für einen Verbund von Dialysegeräten berechnet werden, nämlich für diejenigen Dialysegeräte, die von der jeweiligen RO-Anlage (RO) gespeist werden.

Das System umfasst einen Server, der dazu bestimmt ist, die Sensordaten der RO-Anlage und/oder die Ergebnisdaten der Analyseeinrichtung zu empfangen und der weiterhin dazu bestimmt ist, die Ergebnisdaten zum Zwecke der Steuerung an die RO-Anlage und/oder an diejenigen medizintechnischen Geräte zu übermitteln, die an die RO-Anlage angeschlossen sind. Gegebenenfalls können die Ergebnisdaten noch an weitere Geräte übermittelt werden, die in der jeweiligen Einheit (Klinik/Krankenhausabteilung) in der die RO-Anlage installiert ist, eingebunden sind. Diese Ausführungsform der Erfindung hat den Vorteil, dass der Server in der Cloud ausgebildet sein kann und damit stets ausreichende technische Ressourcen (wie z.B. Prozessorleistung, Speicherkapazität, Implementierung von Applikationen z.B. zum Zwecke der Auswertung) bereitgestellt werden können. Zudem kann dadurch eine zentrale Verarbeitung und eine Aggregation von Daten ermöglicht werden, wobei die erzeugten Daten schnell und frühzeitig über eine Netzwerkverbindung bzw. entsprechende Steuerschnittstellen an die dezentralen Clients (z.B. medizintechnischen Geräte, RO-Anlage) weitergeleitet werden können.

Ein weiterer wichtiger technischer Vorteil ist darin zu sehen, dass die RO-Anlage und/oder z.B. das Dialysegerät direkt auf Grundlage der Ergebnisdaten angesteuert und/oder geregelt werden können. Indizieren die Ergebnisdaten beispielsweise einen Mangel an der Wasserqualität, so kann dies frühestmöglich und direkt an die Empfangsgeräte, also z.B. die Dialysegeräte weitergeleitet werden, um diese unmittelbar vom RO-Wassersystem abzukoppeln bzw. durch einen anderen Anschluss zu ersetzen, falls möglich. Zudem kann die Auswerteeinrichtung auf dem Server auf eine dynamisch auch während des Betriebs anpassbare Regelbasis zugreifen, in der Regeln vorgehalten sind, die z.B. definieren, dass im Fehlerfall Warnmeldungen erzeugt und an unterschiedliche computer-basierte oder elektronische Empfangskonten (z.B. mobile Endgeräte eines Stationsarztes oder Computer im Schwesternzimmer) versendet werden, um rasche Maßnahmen zu ermöglichen.

Der Server mit der Auswerteapplikation kann zudem weitere Daten von anderen Datenquellen empfangen, wie z.B. von einer Wasserzufuhreinheit, die dazu bestimmt ist, der RO-Anlage Wasser zuzuführen, oder von anderen an die RO-Anlage angeschlossenen oder davon versorgten oder damit betriebenen Geräten. Die Wasserzufuhreinheit kann mit einer Messeinheit ausgebildet sein, um Wasserverbrauchsdaten zu erfassen und an den Server zu übermitteln. Der Server kann gemäß einer bevorzugten Ausführungsform der Erfindung ein erstes Ergebnis bereits auf Basis der übermittelten Wasserverbrauchsdaten und/oder Sensordaten bereitstellen. Dazu kann auf dem Server eine Auswerteeinheit bereitgestellt sein, die die empfangenen Daten auf Basis eines hinterlegten Regeldatensatzes auswertet. Der Regeldatensatz kann auch während des Betriebs des Systems dynamisch geändert werden.

Eine Regel kann z.B. lauten: "Wenn die Sensordaten einen vordefinierbaren Schwellenwert unter- oder überschreiten, dann ist die Wasserqualität nicht ausreichend" oder "Wenn die Sensordaten in einem vordefinierbaren Intervall liegen und die Wasserverbrauchsdaten unter einem Schwellenwert liegen, dann ist die Wasserqualität ausreichend". Das derart ermittelte Vorergebnis kann entweder auf dem Server über eine Benutzerschnittstelle ausgegeben werden (z.B. im ersten Beispielfall oben, als Warnmeldung) und/oder an die RO-Anlage oder weitere Geräte weitergeleitet werden.

Dieses Vorergebnis basiert jedoch lediglich auf Messwerten und Sensordaten. Um das Vorergebnis zu validieren, ist eine weitere Analyse vorgesehen. Dazu wird eine Wasserprobe in der Analyseeinrichtung analysiert. Daraufhin können Ergebnisdaten bereitgestellt werden. Diese Ergebnisdaten können nun an den Server übermittelt werden, um das Vorergebnis zu validieren oder zu falsifizieren. Je nachdem werden die Ergebnisdaten wieder auf einer Benutzerschnittstelle des Servers ausgegeben und/oder an die RO-Anlage oder weitere Geräte weitergeleitet. Letzteres erfolgt insbesondere dann, wenn die Qualität als nicht ausreichend bewertet worden ist, um möglichst rasch Gegenmaßnahmen zu ergreifen und insbesondere die Dialysegeräte vom Wassernetz zu entkoppeln. Damit kann zum einen die Qualität der Auswertung verbessert werden und zum anderen kann eine Aussage über die Qualität auch unabhängig von einer labortechnischen Analyse bereitgestellt werden. Die RO-Anlage kann somit vorteilhafterweise wesentlich engmaschiger überwacht werden. Des Weiteren kann umgekehrt auch das Analyseergebnis, das von der Laboranalyse stammt, möglicherweise durch die Sensordaten und/oder Verbrauchsdaten spezifiziert werden bzw. es kann gegebenenfalls sogar eine Ursache eingegrenzt oder angegeben werden. Die Applikation zur Auswertung und Validierung - wie vorstehend beschrieben - muss nicht zwangsweise auf dem Server ausgeführt werden, sondern kann auch ausgelagert werden, beispielsweise direkt auf die RO-Anlage oder auf die Analyseeinrichtung.

In einer weiteren vorteilhaften Ausführungsform der Erfindung umfasst das System weiterhin eine Wasserzufuhreinheit, die zur Zuführung von Wasser in die RO-Anlage bestimmt ist und wobei die Wasserzufuhreinheit eine Messeinheit zur Messung von Wasserverbrauchsdaten umfasst und wobei die Messeinheit eine Busschnittstelle umfasst, um die erfassten Wasserverbrauchsdaten zu versenden (insbesondere bevorzugt an den Server). Mit diesen Daten können umfassendere Auswertungsprozesse gestartet und umfangreichere Ergebnisse bereitgestellt werden. So kann etwa ein Analyseergebnis in zeitlicher Hinsicht den Wasserverbrauchsdaten zugeordnet werden, um eine weiterreichende Aussage bereitstellen zu können. Für Verbrauchswerte, wie z.B. Wasserverbrauch pro Stunde/Tag/Woche/Behandlung können Grenzwerte definiert werden. Wird ein solcher Grenzwert überschritten, kommt es zu einer Meldung. Bis dahin ist es unklar, warum es zu einer Überschreitung kam. Liegen nun weitere Wasserqualitätsdaten vor, wie z.B. die Leitfähigkeit des Rohwassers bzw. Speisewassers und zeigt auch dieser Wert eine Grenzwertüberschreitung, so kann mit dieser intelligenten Verknüpfung bereits die Ursache eingegrenzt/bestimmt werden.

In einer weiteren vorteilhaften Ausführungsform der Erfindung werden die Ergebnisdaten in elektronischer Form in einem vordefinierbaren, standardisierten Format erfasst. Damit können alle Ergebnisdaten einheitlich auf dem Server verarbeitet und/oder auf der RO-Anlage empfangen werden; selbst dann, wenn die Analyseeinrichtungen von unterschiedlichen Betreibern mit unterschiedlichen Verfahren und/oder Applikationen betrieben werden. Dies erhöht die Kompatibilität des Systems und angeschlossener Systeme.

Die von der Sensoreinheit erfassten Sensordaten umfassen Parameter, die die korrekte Funktionsweise der RO-Anlage repräsentieren. Die Parameter umfassen solche zur Leitfähigkeit des Wassers, insbesondere werden hier zwei unterschiedliche Parameter erfasst, nämlich vor und nach der Membran (Speisewasserleitfähigkeit, Pemeatleitfähigkeit), sowie Parameter zum Rückhaltevermögen. Das Rückhaltevermögen R kann beispielsweise mit Hilfe der Leitfähigkeit des Speisewassers cSP und der Leitfähigkeit des Permeats cP wie folgt ermittelt werden:

$$R\ [\%] = (cSp - cP)/cSp * 100\ .$$

In alternativen Ausführungsformen der Erfindung können noch weitere Parameter erfasst werden, um den Aussagegehalt der Analyse oder der Auswertung auf dem Server zu erhöhen (z.B. Strom- und Wasserverbrauch der Wasseraufbereitungsanlage, Temperatur des Wassers, Wasserhärte, Chlorkonzentration).

Die Aufgabe wird weiterhin gelöst durch eine RO-Anlage zur Herstellung von Reinstwasser mit einer Sensoreinheit zur Erfassung von Sensordaten und mit einer elektronischen Datenschnittstelle, die zur Anwendung in einem vorstehend beschriebenen Sicherheitssystem bestimmt ist.

Beschrieben wird weiterhin eine Analyseeinrichtung, die z.B. in einem Labor mit zumindest einer labortechnischen Vorrichtung zur Untersuchung bzw. Analyse der Wasserqualität einer Wasserprobe der RO-Anlage in Hinblick auf Sicherheitserfordernisse an das Reinstwasser (z.B. in Bezug auf Kontaminationen) ausgebildet sein kann und wobei die Analyseeinrichtung dazu bestimmt ist, elektronische Ergebnisdaten in Antwort auf die Analyse der Wasserprobe zu erzeugen, wobei die Analyseeinrichtung weiterhin mit einer Analyseschnittstelle ausgebildet ist, um die erzeugten Ergebnisdaten in elektronischer Form an eine externe Instanz außerhalb der Analyseeinrichtung zu versenden und wobei die Analyseeinrichtung zur Anwendung in einem Sicherheitssystem nach einem der vorstehenden Aspekte bestimmt ist.

Die Aufgabe wird weiterhin gelöst durch einen Server zur koordinierten Verarbeitung von Sicherheitsdaten einer RO-Anlage, die für zumindest ein medizintechnisches Gerät, insbesondere ein Dialysegerät, betrieben wird, wobei der Server zur Anwendung in einem Sicherheitssystem wie oben beschrieben bestimmt ist. Der Server ist ausgebildet mit:

- einer elektronischen Datenschnittstelle zum Austausch von digitalen und/oder analogen Daten, um die von der Sensoreinheit erfassten Sensordaten zu empfangen;
- einer Analyseschnittstelle, um die von der Analyseeinrichtung erzeugten Ergebnisdaten in elektronischer Form zu empfangen.

In einer vorteilhaften Ausführungsform der Erfindung ist der Server weiterhin mit einem Speicher ausgebildet zur Speicherung der empfangenen Daten und/oder interagiert mit einer Datenbank und/oder umfasst eine Verarbeitungseinheit zur spezifischen Verarbeitung der empfangenen Daten. Damit können auch historische Daten verarbeitet werden.

Die Aufgabe wird weiterhin gelöst durch ein Verfahren gemäß dem beiliegenden Verfahrensanspruch.

In einem Aspekt kann das Verfahren zur sicherheitstechnischen Überprüfung einer RO-Anlage, die für einen Betrieb für zumindest ein medizintechnisches Gerät, nämlich ein Dialysegerät, ausgebildet sein, mit folgenden Verfahrensschritten:

- Erfassen von Sensordaten während des Betriebs der RO-Anlage, die zur Herstellung von Reinstwasser bestimmt;
- Versenden der erfassten Sensordaten an einen externen Kommunikationspartner in elektronischer Form. Der externe Kommunikationspartner ist somit außerhalb der RO-Anlage und außerhalb des RO-Anlagensystems angeordnet, so dass eine von der RO-Anlage unabhängige Prüfung sichergestellt werden kann.

In einer bevorzugten Ausführungsform der Erfindung kann nach dem Versenden der Sensordaten oder parallel dazu auf einem Laborsystem ein Analysieren einer Wasserprobe der RO-Anlage in Hinblick auf Sicherheitserfordernisse erfolgen, um auf Basis der Analyse Ergebnisdaten zu erzeugen. Die erzeugten Ergebnisdaten können in elektronischer Form und insbesondere zur Steuerung der RO-Anlage und/oder des Dialysegerätes an die vorstehend genannten Geräte gesendet werden. Alternativ kann die RO-Anlage und/oder das Dialysegerät auf Basis der Ergebnisdaten auch geregelt werden.

In einer bevorzugten Ausführungsform der Erfindung erfolgt das Versenden der erfassten Sensordaten kontinuierlich oder zeitgesteuert während des Betriebs der RO-Anlage und/oder nach vordefinierbaren Ereignissen.

Die Sensordaten und die Ergebnisdaten werden an einen Server zur zentralen Verarbeitung zugeführt und dort gespeichert und insbesondere einer RO-Anlagen übergreifenden statistischen Auswertung zugeführt.

In einer weiteren, bevorzugten Ausführungsform der Erfindung werden die Ergebnisdaten direkt an das mindestens eine medizintechnische Gerät zur Steuerung derselben verwendet, um gegebenenfalls lokal eine Notunterbrechung oder ein Not-Aus auslösen zu können.

Die Erfindung bezieht sich auch auf ein Computerprogrammprodukt, das ein Computerprogramm umfasst. Das Computerprogramm umfasst Softwarecode, der zur Ausführung des oben beschriebenen Verfahrens bestimmt ist. Das Computerprogrammprodukt kann in Software oder in Hardware implementiert sein und zusätzlich zu dem Computerprogramm noch eine Bedienungsanleitung, einen Datenträger und/oder eine Verpackung umfassen. Insbesondere werden die Verfahrensschritte des Erfassens, des Versendens, des Erzeugens von Ergebnisdaten und deren Versendung durch Software getriggert und/oder ausgeführt. Das Analysieren der Probe kann dagegen mehrere Arbeitsschritte umfassen, die mitunter eine menschliche Bedienung erfordern können, während das Erzeugen der Ergebnisdaten wiederrum vollautomatisch erfolgen kann.

Im Folgenden werden die in dieser Anmeldung verwendeten Begrifflichkeiten definiert.

Die RO-Anlage ist eine RO-Anlage zur Erzeugung von Reinstwasser und wird mit zumindest einem medizinischen Gerät angewendet, um selbiges mit Reinstwasser. zu versorgen. Sie kann von daher auch als medizintechnisches Gerät bezeichnet werden. Die RO-Anlage kann eine elektronische Verarbeitungseinheit (z.B. in Form von einer CPU, einem FPGA, einem Mikroprozessor etc.) umfassen. Die Sensoreinheit kann auf der elektronischen Verarbeitungseinheit implementiert sein. Die Norm ISO 23500:2014 definiert die Anforderungen an Dialysewasser (Reinstwasser), die ein Betreiber einer entsprechenden Anlage einzuhalten hat. Dabei greift sie auf die Norm ISO 13959:2014 "Water for haemodialysis and related therapies" zurück, die für einen Hersteller dieser Anlagen Anwendung findet und die folgende Anforderungen an die mikrobiologische und chemische Qualität von Dialysewasser vorgibt:

| | Medium | Grenzwerte | |
|---|---|---|---|
| | | Gesamtkolonienzahl KbE/ml | Endotoxin-Konzentration EU/ml |
| | | | |

(fortgesetzt)

| | Medium | Grenzwerte | |
|---|---|---|---|
| | | Gesamtkolonienzahl KbE/ml | Endotoxin-Konzentration EU/ml |
| ISO 13959: 2014 Water for haemodialysis and related therapies | Dialysewasser | < 100 (AL*=50) | <0,25 (AL*=0,125) |
| *AL=Action level. ISO 13959:2014: Konzentration, ab der Schritte unternommen werden sollen, um den Trend zu höheren, inakzeptalen Werten zu unterbrechen. Der Wert liegt überlicherweise bei 50% des Grenzwertes. | | | |

| | Parameter mit bewiesener Toxität in der Dialyse | Grenzwert [mg/l] | Elektrolyte | Grenzwert [mg/l] | Spurenelemente | Grenzwert [mg/l] |
|---|---|---|---|---|---|---|
| ISO 1359: 2014 | Aluminium | 0,01 | Calcium | 2 | Antimon | 0,006 |
| | Blei | 0,005 | Kalium | 8 | Arsen | 0,005 |
| | Flourid | 0,2 | Magnesium | 4 | Barium | 0,1 |
| | Gesamtchlor | 0,1 | Natrium | 70 | Beryllium | 0,0004 |
| | Kuper | 0,1 | | | Cadmium | 0,001 |
| | Nitrat als (N) | 2 | | | Chrom | 0,014 |
| | Sulfat | 100 | | | Quecksilber | 0,0002 |
| | Zink | 0,1 | | | Selen | 0,09 |
| | | | | | Silber | 0,005 |
| | | | | | Thalium | 0,002 |

Die korrekte Funktionsfähigkeit der RO-Anlage wird unter anderem über das Rückhaltevermögen von Natriumchlorid (Kochsalz) definiert, welches - je nach Anforderungsprofil an das Reinstwasser - zwischen 90% und 99,8% liegen soll.

Die RO-Anlage und das Laborsystem mit der Analyseeinrichtung sind auf zwei unterschiedlichen, getrennten Systemen installiert und bereitgestellt. Üblicherweise wird die RO-Anlage in einem Dialysezentrum bereitgestellt (z.B. in einem von dem Dialysebereich getrennten Bereich), in dem auch die Dialysemaschinen betrieben werden. Das Laborsystem hingegen ist in einem externen Labor angeordnet, das sich außerhalb des Dialysezentrums befindet. Dies hat den Hintergrund, dass die ausgeführte Analyse auch unabhängig (und damit unbeeinflusst) vom Betreiber der Anlage ausgeführt werden können. Damit können Interessenskonflikte vermieden werden. Die Analyseeinrichtung und die RO-Anlage kommunizieren über eine Datenverbindung. Die Datenverbindung kann internet-basiert sein und z.B. auf einem Protokoll der http(s)-Protokollfamilie basieren. Die RO-Anlage und das Laborsystem werden auf unterschiedlichen Plattformen und von unterschiedlichen Betreibern betrieben.

Die Analyseeinrichtung kann Bestandteil eines Laborsystems zur Untersuchung von Wasserproben sein. Die Analyseeinrichtung ist der elektronische Bestandteil und dient zur digitalen Datenverarbeitung und zur Kommunikation der Daten an externe Kommunikationsinstanzen. Neben der Analyseeinrichtung umfasst das Laborsystem somit üblicherweise noch zumindest eine labortechnische Vorrichtung bzw. Gerät (wie z.B. ein Leitfähigkeitsmessgerät, einen Ionenchromatographen, einen Massenspektrographen oder ein Atomabsorptionsspektrometer zur quantitativen Bestimmung einzelner Ionen, etc.). Das Laborsystem dient zur Detektion von Kontaminationen des Wassers sowie zur Durchführung einer biologischen und/oder chemischen Analyse sowie von bakteriologischen Untersuchungen. Ein Aspekt der Erfindung bezieht sich somit auf eine umfassende Analyse der Wasserprobe der RO-Anlage; es wird somit nicht nur etwa ein Chlorgehalt geprüft, sondern es werden weitere Untersuchungen ausgeführt, um Kontaminationen des Wassers zu detektieren (Verschmutzungen, biologische Verunreinigungen oder bakteriologische Verunreinigungen etc.). Aus der ausgeführten Analyse mittels der unterschiedlichen oben beispielhaft genannten Geräte und/oder labortechnischen Vorrichtungen wird ein labortechnisches Ergebnis bereitgestellt. Das Ergebnis wird der Analyseeinrichtung zugeführt, die dazu bestimmt ist, daraus automatisch Ergebnisdaten - z.B. in Form einer elektronischen Nachricht - zu erzeugen. Die Ergebnisdaten können zum Versenden an externe Kommunikationspartner vorbereitet werden. Die Ergebnisdaten sollen insbesondere über eine Datenverbindung an die RO-Anlage und/oder an einen Cloud-basierten Server und/oder an die medizintechnischen Geräte versendet werden, um dort gegebenenfalls weitere Maßnahmen einleiten zu können.

Das Netzwerk ist ein elektronisches Netzwerk zur Übertragung der Daten. Es kann mit unterschiedlichen Protokollen betrieben werden. So kann die Verbindung zwischen der RO-Anlage und dem Server als MBUS-System (insbesondere nach dem Standard der Normenreihe EN13757) ausgelegt sein, und die Analyseeinrichtung kommuniziert mit dem Server und/oder mit den medizintechnischen Geräten über ein IP-basiertes Protokoll, z.B. mittels Nachrichten in einer XML-Struktur. Zum Datenaustausch sind die RO-Anlage und/oder die Analyseeinrichtung mit Schnittstellen ausgebildet: Die RO-Anlage mit einer Datenschnittstelle (z.B. mittels einem IP-basierten Protokoll) und die Analyseeinrichtung mit einer Analyseschnittstelle (z.B. HL7); über diese Schnittstelle können auch Daten in einer tabellenartigen Datenstruktur übertragen werden, wie z.B. in den Formaten csv, Microsoft Excel oder OpenOffice Calc oder xml etc.).

Die erzeugten Ergebnisdaten können in Form von Statusmeldungen (nicht ausreichende Wasserqualität - ausreichende Wasserqualität) oder in Form von umfangreicheren Nachrichtenpaketen übermittelt werden, wobei die Nachrichtenpakete weitere Detailangaben zur Analyse umfassen. Sie können auch Metadaten umfassen, wie z.B. einen Zeitstempel, einen Zustand der Probe, die Dauer der Untersuchung etc.

Wie vorstehend beschrieben, umfasst das System einen - vorzugsweise Cloud-basierten - Server. In dem Server werden die erfassten und erzeugten Daten aggregiert und vorzugsweise gespeichert. Dazu kann ein Zugriff auf eine angeschlossene Datenbank vorgesehen sein. Der Server kann zur konzertierten Verarbeitung der Daten dienen. "Konzertiert" bezieht sich in diesem Kontext darauf, dass die Ergebnisdaten für einen Verbund von Dialysegeräten berechnet worden sind, nämlich für diejenigen Dialysegeräte, die von der jeweiligen RO-Anlage gespeist werden. Die konzertierte Berechnung kann aber auch lokal auf einem dedizierten Gerät, aber dennoch zentral und für alle Geräte des Verbundes gemeinsam ausgeführt werden.

Auf dem Server kann eine Auswerteeinrichtung in Form einer Auswerteapplikation (Software) oder eine Auswerteschaltung (Hardware) ausgebildet sein. Die Auswerteeinrichtung ist eine elektronische Komponente. Die Auswerteschaltung kann z.B. als ein elektronischer Schaltkreis mit digitalen und /oder analogen Schaltungskomponenten ausgebildet sein, der eine Auswertelogik umfassen kann. Die Auswertelogik dient dazu, auszuwerten, welche Qualitätslevel eine analysierte RO-Anlage einhält, um bei Nicht-Einhaltung eine Warnmeldung auszugeben. Die Warnmeldung kann über entsprechende Datenschnittstellen an die RO-Anlage, an eine Steuereinheit der RO-Anlage und/oder direkt an die medizintechnischen Geräte (Dialysegeräte) ausgegeben werden. Zur unmittelbaren Signalisierung kann eine Ampelfunktion auf einer Benutzerschnittstelle ausgegeben werden (rot für zu geringe Qualität, grün für ausreichende Qualität und gelb für Warnung oder Überschreitung von Aktionsleveln, z.B. je nach Über- oder Unterschreitung betreffender Grenzwerte). Die Auswerteschaltung kann zur Auswertung auf ein Regelwerk zugreifen, das insbesondere in Form von Regeln in einer Datenbank oder in einem Speicher hinterlegt sein kann und eine Policy für die Priorisierung einer Menge Ergebnisdaten definiert. Die Auswerteschaltung kann vorzugsweise automatisch aktiviert werden, wenn neue Ergebnisdaten erzeugt oder übermittelt werden. Die Ergebnisdaten werden vorzugsweise nach einem PUSH-Protokoll an die Empfänger zum Zwecke der Regelung und/oder Steuerung gesendet. Die Empfänger können die RO-Anlage oder das Dialysegerät sein.

Die Auswertung ist vorzugsweise einer bestimmten RO-Anlage zugeordnet. Falls ein zentraler Server eingesetzt wird, der Daten von allen oder ausgewählten RO-Anlagen sammelt (z.B. von allen Anlagen in einer bestimmten geographischen Region oder eines Dialyseverbundes) und auswertet, kann mittels einer statistischen Auswertung auch ein RO-Anlagen übergreifendes Ergebnis bereitgestellt werden. Es ist auch möglich, eine Auswertung auszubilden, die die Meldungen für die Anlagen dann je nach RO-Anlagenidentität auflösen kann (beispielsweise über einen entsprechenden Identifikationshinweis).

Ein wichtiger Aspekt der vorliegenden Lösung ist darin zu sehen, dass die medizintechnischen Einrichtungen des Sicherheitssystems, die die RO-Anlage, das Laborsystem mit der Analyseeinrichtung umfassen und darüber hinaus noch die Wasserzufuhreinheit und/oder die Dialysegeräte umfassen können, über jeweils zwei unterschiedliche Verbindungen verbunden sind:

1. Eine Datenverbindung zum Austausch von elektronischen Nachrichten und digitalen Daten, wie z.B. Sensordaten und/oder Ergebnisdaten und
2. Eine Leitungsverbindung zum Austausch von physikalischen Medien, wie Reinstwasser und/oder einer Wasserprobe.

Ein erhöhtes Maß an Sicherheit kann durch eine zusätzliche Überprüfungsmaßnahme bereitgestellt werden. Diese besteht darin, dass die Sensordaten, die lokal auf der RO-Anlage erfasst werden zunächst ausgewertet werden (lokal, in einer Recheneinheit der RO-Anlage, oder extern in einer Recheneinheit auf dem Server oder auf der Analyseeinrichtung), um ein Vorergebnis bereitzustellen. Dieses wird dann ggf. der Analyseeinrichtung bereitgestellt und wird durch die Analyse der Wasserprobe entweder bestätigt (validiert) oder verworfen(falsifiziert). Damit kann noch früher ein vorläufiges Ergebnis auf der RO-Anlage bereitgestellt werden. Zudem kann die Sicherheit erhöht werden, indem das Vorergebnis nochmals überprüft wird.

Im Folgenden wird die Erfindung für ein Dialysegerät als Beispiel eines medizintechnischen Gerätes beschrieben, z.B. eines Hämodialysegerätes. Für den Fachmann liegt es jedoch auf der Hand, dass die Erfindung ebenso auf andere medizintechnische, computergesteuerte Geräte oder (Fluidmanagement-) Maschinen oder Blutbehandlungsgeräte angewendet oder übertragen werden kann, die Reinstwasser zum Betrieb benötigen. Dies kann z.B. auch auf Peritonealdialysegeräte zutreffen, wenn sie Reinstwasser verarbeiten.

Es ist möglich, dass das Übertragen der Sensordaten von der RO-Anlage und/oder von Verbrauchsdaten der Wasserzufuhreinrichtung oder einer anderen Vorrichtung (z.B. betreffend einen Gasverbrauch, einen Stromverbrauch, einen Verbrauch von Temperaturressourcen zur Erwärmung oder Kühlung etc.) in einem konfigurierbaren Zeitintervall ausgeführt wird, um von der Auswerteapplikation verarbeitet zu werden.

In einer bevorzugten Ausführungsform der Erfindung ist es konfigurierbar, an welche Geräte die Ergebnisdaten gesendet werden sollen. So kann es beispielsweise eingestellt werden, dass die Daten, falls sie eine hochpriorisierte Warnmeldung umfassen (Wasserqualität nicht ausreichend) an eine Überwachungseinheit der Klinik/des Betreibers und an weitere Instanzen gesendet werden und im Gutfall (ausreichende Qualität) nur an den Server und/oder an die betreffende RO-Anlage gesendet werden. Dies hat den Vorteil, dass fehlerfrei betriebene Anlagen nicht mit unnötigen Meldungen belastet werden. Es kann aber auch wünschenswert und konfiguriert sein, dass alle Ergebnistypen stets auf allen Geräten verfügbar sind. Damit hat der Betreiber (der klinischen Einrichtung und/oder der RO-Anlage) stets mit einem Blick automatisch alle Anlagen und Zustände der Anlage im Blick.

Eine weitere Aufgabenlösung besteht in einem Computerprogrammprodukt, das in einen Speicher eines Computers oder eines elektronischen oder medizintechnischen Gerätes geladen oder ladbar ist mit einem Computerprogramm zur Durchführung des oben näher beschriebenen Verfahrens, wenn das Computerprogramm auf dem Computer oder dem elektronischen oder medizintechnischen Gerät ausgeführt wird.

Eine weitere Aufgabenlösung sieht ein Computerprogramm vor zur Durchführung aller Verfahrensschritte des oben näher beschriebenen Verfahrens, wenn das Computerprogramm auf einem Computer, einem elektronischen oder medizintechnischen Gerät ausgeführt wird. Dabei ist es auch möglich, dass das Computerprogramm auf einem für den Computer oder das elektronische oder medizintechnische Gerät lesbaren Medium gespeichert ist.

In der folgenden detaillierten Figurenbeschreibung werden nicht einschränkend zu verstehende Ausführungsbeispiele mit deren Merkmalen und weiteren Vorteilen anhand der Zeichnung besprochen.

## Kurze Beschreibung der Figuren

Fig. 1 zeigt in einer schematischen Darstellung ein erfindungsgemäßes Sicherheitssystem mit einer RO-Anlage zum Betrieb von Dialysegeräten und einem Server und deren Datenaustauch gemäß einer vorteilhaften Ausführungsform der Erfindung.

Fig. 2 ist ein Ablaufdiagramm eines Verfahrens gemäß einer bevorzugten Ausführungsform der Erfindung.

Fig. 3 zeigt auf schematische Weise den Datenaustausch zwischen Dialysegerät und Server und der RO-Anlage gemäß einer Ausführungsform der Erfindung und

Fig. 4 zeigt eine zu der in Fig. 1 alternative Ausführungsform der Erfindung des Sicherheitssystems ohne einen Server.

## Detaillierte Beschreibung der Figuren

Im Folgenden wird die Erfindung anhand von Ausführungsbeispielen unter Bezugnahme auf die Figuren näher beschrieben.

Die Erfindung betrifft ein elektronisches Nachrichtensystem für RO-Anlagen RO, die für Dialysestationen mit zumindest einem Dialysegerät oder anderen medizintechnischen Geräten D betrieben und angewendet werden und die einen Qualitätszustand der RO-Anlage RO kommunizieren.

**Fig. 1** zeigt eine erste Ausführungsform der Erfindung, bei der das System 1 einen Server SV umfasst. Der Server SV kann zumindest teilweise zur Auswertung von Wasserqualitätsdaten ausgebildet sein. Die Auswertung der Wasserqualität basiert auf unterschiedlichen Eingangsgrößen, die von unterschiedlichen Geräten (RO-Anlage RO, Analyseeinrichtung AE, Datenbank DB etc.) bereitgestellt werden.

Dazu wird ein Sicherheitssystem 1 bereitgestellt, das mehrere medizintechnische Einrichtungen umfasst, darunter medizintechnische Geräte mit jeweils elektronischen Komponenten zur Datenverarbeitung und zur Kommunikation.

Die RO-Anlage RO ist zur Herstellung von Reinstwasser bestimmt, welches einem oder - in der Regel - mehreren Dialysegerät(en) D einer Dialysestation zugeführt werden muss, damit diese betrieben werden können. Zur Sicherstellung einer ausreichenden Qualität des zugeführten Reinstwasser (Einhaltung der Grenzwerte von Kontaminationen, z.B. von Aluminium, Chlor, Fluoriden, Nitraten, Sulfaten und/oder Zink - die Grenzwerte für eine jeweilige Maximal Konzentration sind in der Norm ISO 13959:2014 wie oben angegeben definiert) ist die RO-Anlage RO mit einer Sensoreinheit S zur Erfassung von Sensordaten ausgebildet (in Figur 1 beispielhaft mit Sensoren S1, S2, Sn). Darüber hinaus umfasst die RO-Anlage RO eine elektronische Datenschnittstelle RO-S, um die von der Sensoreinheit S erfassten Sensordaten zu versenden.

Die RO-Anlage RO wird von einer Wasserzufuhreinheit W gespeist, die dazu dient, Wasser zuzuführen, damit dieses in der RO-Anlage RO gereinigt bzw. aufbereitet werden kann. Die Wasserzufuhreinheit W umfasst mehrere elektronische Module, wie unter anderem eine Messeinheit M, die dazu bestimmt ist, Wasserverbrauchsdaten 32 zu ermitteln. Dazu können unterschiedliche Messmethoden und Fühler bzw. Signalgeber zum Einsatz kommen. Weiterhin umfasst die Wasserzufuhreinheit W Schnittstellen zur Datenkommunikation, die insbesondere in Form einer MBUS-Schnittstelle MBUS ausgebildet sein kann. Andere medizintechnische Einrichtungen des Systems 1, wie z.B. der Server SV und/oder die Analyseeinrichtung AE können über diese Schnittstelle MBUS mit der Wasserzufuhreinheit W kommunizieren. Damit wird es möglich, dass die Analyseeinrichtung AE Sensordaten direkt von der Wasserzufuhreinheit W erfassen kann. Dies hat den vorteilhaften Effekt, dass die Analyseeinrichtung AE eine umfassendere Auswertung ausführen kann, die insbesondere die Wasserverbrauchsdaten 32 und ggf. weitere Sensordaten, die auf der Wasserzufuhreinheit W erfasst werden, für die Berechnung der Ergebnisdaten berücksichtigt.

Die auf der Wasserzufuhreinheit W erfassten Sensordaten können in einer weiteren vorteilhaften Ausführungsform der Erfindung an die RO-Anlage RO weitergeleitet werden. Dies hat den Vorteil, dass die Sensordaten der Wasserzufuhreinheit W mit den lokal erfassten Sensordaten der RO-Anlage zu einem Vorergebnis berechnet werden können, das an die Analyseeinrichtung zum Zwecke der Validierung gesendet wird. Das Vorergebnis kann auf einer Ausgabeeinheit (z.B. Bildschirm) der Wasserzufuhreinheit W und/oder der RO-Anlage RO zur lokalen Kontrolle ausgegeben werden. Damit können umfassendere Berechnungen für das Vorergebnis ausgeführt werden, die mehr Aussagekraft haben.

Die Analyseeinrichtung AE kann in einem Laborsystem angeordnet sein. Das Laborsystem mit labortechnischen Vorrichtungen ist dazu bestimmt, eine Wasserprobe der RO-Anlage in Hinblick auf Sicherheitserfordernisse und insbesondere auf Kontamination, zu analysieren. Auf Basis des Analyseergebnisses und ggf. unter Berücksichtigung von zusätzlich erfassten Sensordaten (von der Wasserzufuhreinheit W und/oder von der RO-Anlage RO), werden gemäß vorgehaltenen Regeln Ergebnisdaten berechnet bzw. erzeugt. Die Ergebnisdaten werden auch in einem digitalen Format, insbesondere in einem Ergebnisformat bereitgestellt. Dabei kann es sich um eine konfigurierbare Datenstruktur, insbesondere nach dem XML-Format handeln. Die Analyseeinrichtung AE umfasst weiterhin eine Analyseschnittstelle AE-S, um die erzeugten Ergebnisdaten in elektronischer Form an externe Kommunikationspartner (insbesondere an die RO-Anlage RO und/oder an die angeschlossenen Dialysegeräte D) zu versenden.

Die Einrichtungen und Geräte des Sicherheitssystems 1 sind über ein Netzwerk NW miteinander verbunden.

Wie in Fig. 1 angedeutet, sind an eine RO-Anlage typischerweise mehrere Dialysegeräte D und/oder weitere Einrichtungen angeschlossen Dies soll in Fig. 1 durch die beiden beispielhaft gezeigten Geräte D1, Dn repräsentiert sein.

Das Sicherheitssystem 1 umfasst in einer in Fig. 1 dargestellten (ersten) bevorzugten Ausführungsform der Erfindung einen Server SV. Dieser ist über Netzwerkschnittstellen über ein technisches Kommunikationsnetzwerk NW vorzugsweise zentral zugreifbar und kann als Cloud-Server ausgebildet sein. Der Server SV steht mit den angeschlossenen Geräten in Datenaustausch, insbesondere mit der RO-Anlage RO, mit den medizintechnischen Geräten D, mit der Analyseeinrichtung AE und gegebenenfalls mit einer Datenbank DB. In dieser ersten Ausführungsform der Erfindung ist auf dem Server SV in einem Prozessor P eine Auswerteapplikation oder eine Auswertefunktionalität implementiert, die zur Auswertung der detektierten Daten bestimmt ist. Insbesondere werden die Ergebnisdaten und die Sensordaten und gegebenenfalls historische Daten aus einer Datenbank DB nach vordefinierbaren Regeln verarbeitet, um eine Ergebnisnachricht über den Qualitätszustand des von der RO-Anlage bereitgestellten Wasser zu indizieren. Die Ergebnisnachricht kann vorzugsweise zur Steuerung der RO-Anlage RO und/oder der angeschlossenen Dialysegeräte D verwendet werden. Damit können die relevanten Ergebnisse unmittelbar lokal am Point of use bereitgestellt werden.

In der Datenbank können in einer bevorzugten Ausführungsform der Erfindung des Weiteren auch konfigurierbare Regeln hinterlegt sein, die spezifizieren, wann die Ergebnisdaten an die jeweiligen Empfänger zu senden sind. Des Weiteren kann - z.B. spezifisch für bestimmte geographische Regionen oder Länder - definiert werden, welche weiteren Funktionen und Nachrichten zusammen mit den Ergebnisdaten in einem Datenpaket an die Empfänger zu senden sind. Bei den Funktionen kann es sich z.B. um Steuerfunktionen für das Dialysegerät und/oder die RO-Anlage (An-Abschalten des Dialysegerätes, Beschränken der Gerätefunktionalität - insbesondere in Abhängigkeit vom Analyseergebnis etc.) und bei den Nachrichten um Erstellen von Warnmeldungen (z.B. auf der RO-Anlage, dass die Wasserqualität nicht den geforderten Sicherheitsanforderungen genügt mit Hinweisen auf Grenzwertüberschreitungen) handeln. Die Regeln können in einer Konfigurationsphase dediziert für die jeweiligen Empfänger der Ergebnisdaten (oder Datenpakte) oder von Empfängergruppen unterschiedliche spezifiziert werden. Damit wird vorteilhafterweise eine wichtige weitere Flexibilität erreicht.

Der Server SV und die darauf implementierte Auswerteapplikation kann vorzugsweise als Web-Plattform und Browser-basiert bereitgestellt werden. Der Server SV kann für weitere Berechnungen, z.B. statistische Auswertungen, auf einen lokalen Speicher MEM zugreifen und/oder die berechneten oder eingelesenen Daten dort speichern.

Wie oben bereits kurz erläutert, ist die Analyseeinrichtung AE dazu bestimmt, aus dem Laborbericht oder den Laborergebnissen Ergebnisdaten nach einem vordefinierten Format zu erzeugen, um diese an externe Kommunikationspartner zu übertragen.

In Fig. 1 sollen die gestrichelt gekennzeichneten Pfeile (von der Wasserzufuhreinheit W an die RO-Anlage RO und von der RO-Anlage an die Analyseeinrichtung AE) indizieren, dass es sich hier nicht um einen Datentransfer, sondern um den Transfer von physikalischen Medien handelt, also im ersten Fall wird Wasser an die RO-Anlage RO transferiert und im zweiten Fall wird eine Wasserprobe an die Analyseeinrichtung AE übermittelt. Die anderen Pfeile kennzeichnen den elektronischen Austausch von analogen und/oder digitalen Daten.

Grundsätzlich kann das System in zwei Ausführungsvarianten betrieben werden.

Wie oben beschrieben, ist in einer ersten, in Fig. 1 gezeigten Ausführungsvariante dem System 1 ein zentraler Server SV zugeschaltet. Auf dem Server SV ist dann die Auswerteapplikation zur Auswertung der detektierten Daten implementiert. Der Server SV ist vorzugsweise Cloud-basiert und über IP-Protokoll basierte Schnittstellen (z.B. TCP/IP) SV-S1, SV-S2 zugreifbar. In dieser Ausführungsform der Erfindung werden die ausgetauschten Daten von dem jeweiligen Sender (z.B. RO-Anlage RO, Analyseeinrichtung AE) zunächst an den zentralen Server SV geleitet, der dann die empfangenen Daten entweder direkt oder in vorverarbeiteter Form an den jeweiligen Empfänger sendet (z.B. RO-Anlage RO, Analyseeinrichtung AE). Dazu können auch die Daten von der Wasserzufuhreinheit W und/oder die Daten der Dialysegeräte D über die Schnittstellen SV-S1, SV-S2 kommunizieren (in Fig. 1 nicht explizit dargestellt). Damit agiert der Server SV in diesem Ausführungsbeispiel als Proxy oder Mittlerknoten (intermediary node) in der Kette zwischen Datenquelle und Datensenke. Diese Ausführungsform der Erfindung hat den Vorteil, dass alle Daten auf dem Server SV aggregiert werden können, um eine weitere Auswertung und Verarbeitung ausführen zu können. So können insbesondere historische Datensätze mit aktuellen Datensätzen abgeglichen werden, um weitergehende Aussagen bereitstellen zu können (z.B. "In 80% der Fälle, in denen die Ergebnisdaten keine ausreichende Wasserqualität indizieren, wurde die Probe von einer Gruppe von RO-Anlagen entnommen, die in einer bestimmte geografischen Region lokalisiert sind" oder "in 90% der Fälle, in denen die Ergebnisdaten keine ausreichende Wasserqualität indizieren, wurde die Probe in einer bestimmten Zeitphase entnommen"). Insbesondere kann eine statistische Auswertung ausgeführt werden, die RO-Anlagen übergreifend ist. Zudem können dabei ermittelte Referenzdaten auf anderen RO-Anlagen zum Zwecke des Vergleichs/der Referenz bereitgestellt werden. Weiterhin können auch manuell eingegebene Daten in Bezug auf die RO-Anlage RO im Rahmen der Auswerteapplikation berücksichtigt werden. Zum Zugriff und zur Speicherung der Daten kann der Speicher MEM verwendet werden.

Beschrieben ist auch ein System, bei dem kein zentraler Server vorgesehen ist. In diesem Fall interagiert die RO-Anlage und/oder die Wasserzufuhreinheit W und/oder das Dialysegerät D direkt mit der Analyseeinrichtung AE und umgekehrt. Diese zweite Ausführungsform soll in Fig. 1 dadurch repräsentiert sein, dass zumindest die RO-Anlage RO direkt (ohne Vermittlung durch den Server SV) mit der Analyseeinrichtung AE kommuniziert, was mit dem Pfeil zwischen den entsprechenden Schnittstellen RO-S, AE-S, der ohne Vermittlung durch den Server SV verläuft, gekennzeichnet ist. In diesem Fall kann auf der Analyseeinrichtung AE zumindest teilweise die Auswerteapplikation zur Auswertung der Daten und zur Berechnung der Ergebnisnachricht bereitgestellt werden. Teilweise kann die Applikation auch auf anderen elektronischen Geräten implementiert sein. Die Ergebnisdaten oder die Ergebnisnachricht umfassen dann einen Steuerdatensatz, der zur Steuerung des jeweiligen Gerätes ausgebildet ist. Im Fehlerfall (keine ausreichende Reinstwasserqualität) kann der Steuerdatensatz einen Abschnitt umfassen, der z.B. die Ausgabe einer Warnmeldung und/oder eine Abschaltung der RO-Anlage RO triggert. Des Weiteren kann der Steuerdatensatz ein Benachrichtigungsfeld umfassen, das eine Benachrichtigung von weiteren Instanzen oder Geräten auslöst. Diese Benachrichtigung soll insbesondere dann zur Ausführung kommen, wenn der Steuerdatensatz an den externen Kommunikationspartner (z.B. an die RO-Anlage RO) übertragen worden ist. So können z.B. automatisch Warnmeldungen direkt und lokal an den Dialysegeräten D ausgelöst werden, die an die RO-Anlage RO angeschlossen sind. Dies hat den Vorteil, dass in sicherheitskritischen Fällen die relevanten Informationen unmittelbar lokal zur Verfügung gestellt werden und somit notwendige Maßnahmen direkt eingeleitet werden können, ohne dass zwischengeschaltete

Instanzen informiert werden müssen. In einer vorteilhaften Variante ist es vorgesehen, dass die Ergebnisdaten oder die Ergebnisnachricht von einem Anwender (z.B. einem Laborarzt) freigegeben werden muss, bevor sie an weitere Geräte und Instanzen weitergeleitet wird. Dies kann über ein bereitgestelltes Feld und eine darauf erfasste Benutzereingabe ausgeführt werden. Die Freigabe kann an unterschiedliche Rollen des Anwenders (mit spezifischen Berechtigungen) geknüpft sein.

Diese Ausführungsform wird weiter unten anhand von Fig. 4 näher erläutert.

**Fig. 2** zeigt den Ablauf des Verfahrens gemäß einer bevorzugten Ausführungsform der Erfindung. Nach dem Start des Verfahrens zur sicherheitstechnischen Qualitätsprüfung der RO-Anlage RO und damit des bestimmungsgemäßen Betriebs von angeschlossenen Dialysegeräten D, werden in Schritt 100 Sensordaten während des Betriebs der RO-Anlage RO erfasst. Dies erfolgt vorzugsweise in vordefinierbaren Zeitabständen, nach vorkonfigurierbaren Ereignissen (z.B. bei Anschluss eines weiteren Dialysegerätes D und/oder nach Durchführung einer bestimmten Anzahl von Dialysen) und/oder kontinuierlich während des RO-Betriebs. In Schritt 200 werden die auf der RO-Anlage RO und/oder auf der Wasserzufuhreinheit W erfassten Sensordaten an einen externen Kommunikationspartner (außerhalb der RO-Anlage) in elektronischer Form übermittelt. Je nach einer der beiden oben beschriebenen Ausführungsvarianten werden die Sensordaten an den Server SV oder an die Analyseeinrichtung AE übermittelt. Die Analyseeinrichtung AE empfängt zudem die Wasserprobe und analysiert diese, um daraufhin Ergebnisdaten bereitstellen zu können. Dies erfolgt in Schritt 300. Im anschließenden Schritt 400 werden die erzeugten Ergebnisdaten in elektronischer Form zur Steuerung der RO-Anlage und/oder des medizintechnischen Gerätes D entweder direkt an die jeweiligen Geräte RO, D und/oder an den Server SV übermittelt. Vom Server SV werden sie dann vermittelt weitergeleitet und können zudem dort zentral gespeichert werden. Damit kann eine erste RO-Anlage RO auch auf Referenzdaten von anderen zweiten RO-Anlagen in vergleichender Form zugreifen. Danach kann das Verfahren iterativ ausgeführt oder beendet werden.

**Fig. 3** zeigt ein Sequenzdiagramm mit den beiden oben geschilderten unterschiedlichen Varianten zum Datenaustausch zwischen den elektronischen Einheiten des Systems 1:

1. Mit zentralem Server SV und darauf implementierter Auswerteapplikation (Strich-Punkt Linien);
2. Ohne Server (durchgezogenen Linien). Hier interagieren die RO-Anlage und die Dialysegeräte D und die Messeinheit M direkt mit der Auswerteapplikation, die in diesem Fall auf der Analyseeinrichtung AE implementiert ist.

Während des Betriebs der RO-Anlage werden lokal Sensordaten 31 erfasst und von dort direkt an die Analyseeinrichtung AE übermittelt (durchgezogener Pfeil). Alternativ werden die Sensordaten 31 zunächst an den Server SV und vor dort an die Analyseeinrichtung AE übermittelt (in Fig. 3 strich-gepunktet dargestellt). Parallel oder gleichzeitig werden auf der Messeinheit M der Wasserzufuhreinheit W oder einer anderen Vorrichtung Wasserverbrauchsdaten 32 erfasst, die in der ersten Variante an den Server SV zum Zwecke der Auswertung übermittelt werden (in Fig. 3 strich-gepunktet dargestellt). Alternativ oder kumulativ können die Wasserverbrauchsdaten 32 auch an die Analyseeinrichtung AE übermittelt werden (durchgezogene Linie). In diesem Fall ist auf der Analyseeinrichtung AE eine Applikation zur Auswertung der Daten implementiert, sodass die entsprechende Funktionalität des Servers SV in diesem Fall auf die Analyseeinrichtung AE transferiert wird (schematisch in Fig. 4 dargestellt). Die Analyseeinrichtung AE erzeugt auf Basis der ausgeführten Laboruntersuchung bzw. Analyse einen Ergebnisdatensatz 33, der nun ebenfalls entweder direkt an die medizintechnischen Geräte RO, D, W übermittelt wird (durchgezogene Linien) - oder in der anderen Ausführungsvariante über Vermittlung und/oder Speicherung des Servers SV, der die Daten dann in verarbeiteter oder unverarbeiteter Form an die Empfänger RO, D W weiterleitet (in Fig. 3 strich-gepunktet dargestellt). Die Verarbeitung und Auswertung auf dem Server SV kann weitere Prozessschritte umfassen, wie vorstehend beschrieben, wie z.B. eine statistische Auswertung oder einen Abgleich mit historischen Daten. Das weitere Ergebnis dieser Prozessschritte ist in Fig. 3 mit dem Bezugszeichen 33' gekennzeichnet und kann an die jeweiligen lokalen Instanzen RO, W, D übermittelt werden.

**Fig. 4** zeigt schematisch ein Beispiel, bei dem das System ohne Server betrieben wird. Die gestichelten Linien (W -> RO, RO -> AE) repräsentieren - wie auch in Fig. 1 - keinen Datenaustausch, sondern die Übermittlung von physikalischen Produkten (Wasser, Reinstwasser). Bezüglich des Datenaustausches interagieren die RO-Anlage RO und die Analyseeinrichtung AE und gegebenenfalls die Wasserzufuhreinheit W direkt miteinander über ein Netzwerk, das z.B. TCP/IP-basiert ein kann. In dieser Ausführungsform der Erfindung ist auf der Analyseeinrichtung AE die Funktionalität implementiert, die bei dem ersten Ausführungsbeispiel auf dem Server SV implementiert war. Die Daten der Wasserzufuhreinheit W bzw. deren Messeinheit M, die Sensordaten der RO-Anlage RO und ggf. Daten der Dialysegeräte D werden direkt an die Analyseeinrichtung AE gesendet und dort verarbeitet. Es ist auch möglich, dass die Wasserzufuhreinheit W über eine Datenschnittstelle mit der RO-Anlage RO verbunden ist. Die auf der Wasserzufuhreinheit W erfassten Daten können dann indirekt und über die Vermittlung der RO-Anlage RO an die Analyseeinrichtung AE gesendet werden. Zur Verarbeitung der eingelesenen Daten auf der Analyseeinrichtung AE können Referenzdaten von der Datenbank DB eingelesen werden und umgekehrt die von der Analyseeinrichtung AE erfassten Daten und die verarbeiteten Daten können in der Datenbank DB gespeichert werden. Das Analyseergebnis wird dann entweder an die RO-Anlage übermittelt (Strichpunkt Linie), die dann die Daten an die Wasserzufuhreinheit W und an das Dialysegerät D weiterleitet (ebenfalls Strichpunkt Linie) oder die Daten können von der Analyseeinrichtung AE direkt an das Dialysegerät D und/oder an die Wasserzufuhreinheit W zum Zwecke der Steuerung gesendet werden (diese Ausführung ist in Fig. 4 mit dem Pfeil gekennzeichnet, der durchgezogen ist).

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren. Es liegt somit z.B. ebenso im Rahmen der Erfindung, alternativ oder kumulativ zum Server SV andere zentrale Einheiten, wie z.B. eine Datenbank DB bereitzustellen. Ebenso können an die RO-Anlage RO neben den Dialysegeräten D noch weitere medizintechnische und/oder Rechner-gestützte Geräte (so auch mobile Endgeräte) angeschlossen sein, auf denen die Ergebnisdaten ausgegeben werden. Für einen Fachmann ist es insbesondere offensichtlich, dass die Erfindung nicht nur für Dialysegeräte angewendet werden kann, sondern auch für andere medizintechnische Geräte D, für deren Betrieb Reinstwasser aus einer RO-Anlage RO erforderlich ist. So kann z.B. die Überwachung der Qualität des Reinstwassers auch für Sterilisations- und Waschprozesse zur Sterilisation von klinischen Besteck verwendet werden.

Des Weiteren können die Bauteile bzw. Module des Sicherheitssystems zur Überwachung der Qualität des Reinstwassers auf mehrere physikalische Produkte verteilt realisiert werden. So liegt es z.B. im Rahmen der Erfindung, dass eine Applikation zur Auswertung der Ergebnisdaten vollständig oder teilweise auf der Analyseeinrichtung AE angeordnet ist oder dass die Applikation vollständig oder teilweise auf dem Server SV implementiert ist. Zudem können Abschnitte des Computerprogramms zur Ausführung des Verfahrens auch direkt auf den medizintechnischen Geräten D, RO implementiert sein.

Der Schutzbereich der vorliegenden Erfindung ist durch die Ansprüche gegeben und wird durch die in der Beschreibung erläuterten oder den Figuren gezeigten Merkmale nicht beschränkt.

BEZUGSZEICHEN

| | |
|---|---|
| D | Medizintechnisches Gerät, insbesondere Dialysegerät |
| SV | Server |
| P | Auswerteeinrichtung |
| MEM | Speicher |
| RO | Umkehrosmose Anlage, kurz: RO-Anlage |
| AE | Analyseeinrichtung |
| AE-S | Analyseschnittstelle der Analyseeinrichtung |
| RO-S | Datenschnittstelle der RO-Anlage |
| W | Wasserzufuhreinheit |
| M | Messeinheit der Wasserzufuhreinheit |
| MBUS | Busschnittstelle der Wasserzufuhreinheit |

| | |
|---|---|
| 100 | Erfassen von Sensordaten |
| 200 | Versenden von auf der RO-Anlage erfassten Sensordaten |
| 300 | Erzeugen von Ergebnisdaten |
| 400 | Versenden von Ergebnisdaten |

| | |
|---|---|
| DB | Datenbank |
| NW | Netzwerk |

## Patentansprüche

1. Elektronisches Sicherheitssystem für eine RO-Anlage (RO), die für eine Anwendung mit zumindest einem medizintechnischen Gerät (D), nämlich einem Dialysegerät, ausgebildet ist, mit:

   - der RO-Anlage (RO), die zur Herstellung von Reinstwasser bestimmt und mit einer Sensoreinheit (S) zur Erfassung von Sensordaten ausgebildet ist und wobei die RO-Anlage (RO) eine elektronische Datenschnittstelle (RO-S) umfasst, um die von der Sensoreinheit (S) erfassten Sensordaten zu versenden, wobei die von der Sensoreinheit erfassten Sensordaten Parameter umfassen, die die korrekte Funktionsweise der RO-Anlage repräsentieren, nämlich es werden zwei unterschiedliche Parameter vor und nach der Membran erfasst, nämlich die Speisewasserleitfähigkeit und die Permeatleitfähigkeit sowie Parameter zum Rückhaltevermögen;
   - einem Laborsystem mit zumindest einer labortechnischen Vorrichtung und mit einer Analyseeinrichtung (AE), wobei die zumindest eine labortechnische Vorrichtung dazu bestimmt ist, eine Wasserprobe der RO-Anlage in Hinblick auf Sicherheitserfordernisse zu analysieren und wobei das Laborsystem zur Detektion von Kontaminationen des Wassers sowie zur Durchführung einer biologischen und/oder chemischen Analyse sowie von bakteriologischen Untersuchungen dient, und wobei die Analyseeinrichtung (AE) dazu bestimmt ist, auf Basis der Analyse der zumindest einen labortechnischen Vorrichtung, Ergebnisdaten zu erzeugen, wobei die Analyseeinrichtung (AE) weiterhin mit einer Analyseschnittstelle (AE-S) ausgebildet ist, um die erzeugten Ergebnisdaten in elektronischer Form zu versenden; einem Netzwerk (NW) zum Datenaustausch zwischen medizintechnischen Einrichtungen des Sicherheitssystems, nämlich zwischen der RO-Anlage (RO) und der Analyseeinrichtung (AE),

- weiterhin umfassend einen Server mit einer Auswerteeinrichtung, auf dem die erfassten und erzeugten Daten aggregiert werden und der zur konzertierten Verarbeitung der Daten dient, wobei die Ergebnisdaten für einen Verbund von Dialysegeräten berechnet werden, nämlich für diejenigen Dialysegeräte, die von der jeweiligen RO-Anlage (RO) gespeist werden.

**2.** Sicherheitssystem nach Anspruch 1, wobei die RO-Anlage (RO) und das Laborsystem mit der Analyseeinrichtung auf zwei unterschiedlichen, getrennten Systemen installiert und bereitgestellt sind, wobei die RO-Anlage und die Analyseeinrichtung mit einer Datenverbindung, die internet-basiert sein kann, miteinander kommunizieren.

**3.** Sicherheitssystem nach Anspruch 1, wobei der Server (SV) dazu bestimmt ist, die Sensordaten der RO-Anlage (RO) und/oder die Ergebnisdaten der Analyseeinrichtung (AE) zu empfangen und der weiterhin dazu bestimmt ist, die Ergebnisdaten an die RO-Anlage (RO) und/oder an das medizintechnische Gerät (D) zum Zwecke der Steuerung und/oder Regelung zu übermitteln.

**4.** Sicherheitssystem nach einem der vorstehenden Ansprüche, bei dem das System weiterhin eine Wasserzufuhreinheit (W) umfasst, die zur Zuführung von Wasser in die RO-Anlage (RO) bestimmt ist und wobei die Wasserzufuhreinheit (W) eine Messeinheit (M) zur Messung von Wasserverbrauchsdaten umfasst und wobei die Messeinheit (M) eine Busschnittstelle (MBUS) umfasst, um die erfassten Wasserverbrauchsdaten zu versenden.

**5.** Sicherheitssystem nach einem der vorstehenden Ansprüche, bei dem aus den erfassten Sensordaten lokal ein Vorergebnis berechnet wird, das an die Analyseeinrichtung (AE) gesendet wird, um in der Analyseeinrichtung (AE) auf Basis der empfangenen Wasserprobe validiert oder falsifiziert zu werden.

**6.** RO-Anlage (RO) zur Herstellung von Reinstwasser mit einer Sensoreinheit (S) zur Erfassung von Sensordaten und mit einer elektronischen Datenschnittstelle (RO-S), die in einem Sicherheitssystem nach einem der vorstehenden Ansprüche eingesetzt ist.

**7.** Server (SV) zur koordinierten Verarbeitung von Sicherheitsdaten einer RO-Anlage (RO), die für zumindest ein medizintechnisches Gerät (D), nämlich ein Dialysegerät, betrieben wird, wobei der Server (SV) in einem Sicherheitssystem nach den vorstehenden Systemansprüchen verwendet wird, mit:

- einer elektronischen Datenschnittstelle (SV-S1), um die von der Sensoreinheit (S) erfassten Sensordaten zu empfangen;
- einer Analyseschnittstelle (SV-S2), um die von der Analyseeinrichtung erzeugten Ergebnisdaten in elektronischer Form zu empfangen; und umfassend
- eine Auswerteeinrichtung (P) zur weiteren Verarbeitung der empfangenen Daten.

**8.** Server (SV) nach dem unmittelbar vorangehenden Anspruch, bei dem der Server (SV) weiterhin einen Speicher (MEM) umfasst, zur Speicherung der empfangenen Daten und/oder mit einer Datenbank (DB) interagiert.

**9.** Server (SV) nach einem der vorangehenden auf den Server gerichteten Ansprüche, bei dem der Server (SV) weiterhin eine Steuerschnittstelle umfasst, um die RO-Anlage (RO) und/oder das oder die medizintechnischen Geräte (D) auf Basis der Ergebnisdaten zu steuern.

**10.** Verfahren zur sicherheitstechnischen Überprüfung einer RO-Anlage (RO), die für eine Anwendung mit zumindest einem medizintechnischen Gerät (D), nämlich einem Dialysegerät, insbesondere in einem Sicherheitssystem nach einem der Ansprüche 1 bis 5 ausgebildet ist, mit folgenden Verfahrensschritten:

- Erfassen (100) von Sensordaten während des Betriebs der RO-Anlage (RO), die zur Herstellung von Reinstwasser bestimmt ist, wobei die von einer Sensoreinheit erfassten Sensordaten Parameter umfassen, die die korrekte Funktionsweise der RO-Anlage (RO) repräsentieren, nämlich es werden zwei unterschiedliche Parameter vor und nach der Membran erfasst, nämlich die Speisewasserleitfähigkeit und die Permeatleitfähigkeit sowie Parameter zum Rückhaltevermögen;
- Versenden (200) der erfassten Sensordaten an einen externen Kommunikationspartner in elektronischer Form;
- Empfangen von Ergebnisdaten, die eine Analyse einer Wasserprobe der RO-Anlage in Hinblick auf Sicherheitserfordernisse repräsentieren;
- wobei die Sensordaten und die Ergebnisdaten an einen Server (SV) zur zentralen Verarbeitung zugeführt und dort gespeichert werden.

**11.** Verfahren nach dem vorstehenden Verfahrensanspruch, bei dem das Erfassen (100) der Sensordaten kontinuierlich oder zeitgesteuert während des Betriebs der RO-Anlage (RO) und/oder nach vordefinierbaren Ereignissen erfolgt.

**12.** Verfahren nach einem der vorstehenden Verfahrensansprüche, bei dem die Sensordaten und die Ergebnisdaten an den Server (SV) zur zentralen Verarbeitung und insbesondere einer RO-Anlagen übergreifenden statistischen Auswertung zugeführt werden.

**13.** Computerprogrammprodukt mit einem Computerprogramm mit Programmabschnitten zur Durchführung aller Verfahrensschritte des Verfahrens gemäß einem der vorangehenden Verfahrensansprüche, wenn das Computerprogramm auf einem Computer oder einem elektronischen Gerät ausgeführt wird.

## Claims

**1.** Electronic security system for an RO system (RO) designed for use with at least one medical device (D), namely a dialysis machine, comprising:

- the RO system (RO), which is designed to produce ultrapure water and is equipped with a sensor unit (S) for recording sensor data, and wherein the RO system (RO) comprises an electronic data interface (RO-S) for sending the sensor data recorded by the sensor unit (S), wherein the sensor data detected by the sensor unit comprises parameters that represent the correct functioning of the RO system, namely two different parameters are detected before and after the membrane, namely the feed water conductivity and the permeate conductivity , as well as parameters for the retention capacity;
- a laboratory system with at least one laboratory device and an analysis device (AE), wherein the at least one laboratory device is designed to analyze a water sample from the RO system with regard to security requirements, and wherein the laboratory system serves to detect contamination of the water and to perform biological and/or chemical analysis and bacteriological tests, and wherein the analysis device (AE) is designed to generate result data based on the analysis of the at least one laboratory device, wherein the analysis device (AE) is further equipped with an analysis interface (AE-S) in order to send the generated result data in electronic form;
- a network (NW) for data exchange between medical devices of the security system, namely between the RO system (RO) and the analysis device (AE),
- further comprising a server with an evaluation device, on which the received and generated data are aggregated and which serves for the concerted processing of the data, wherein the result data are calculated for a network of dialysis devices, namely for those dialysis devices that are fed by the respective RO system (RO).

**2.** Security system according to claim 1, wherein the RO system (RO) and the laboratory system with the analysis device are installed and provided on two different, separate systems, wherein the RO system and the analysis device communicate with each other via a data connection, which may be internet-based.

**3.** Security system according to claim 1, wherein the server (SV) is designed to receive the sensor data from the RO system (RO) and/or the result data from the analysis device (AE) and is further designed to transmit the result data to the RO system (RO) and/or to the medical device (D) for the purpose of control and/or regulation.

**4.** Security system according to one of the preceding claims, wherein the system further comprises a water supply unit (W) designed to supply water to the RO system (RO), and wherein the water supply unit (W) comprises a measuring unit (M) for measuring water consumption data, and wherein the measuring unit (M) comprises a bus interface (MBUS) for sending the recorded water consumption data.

**5.** Security system according to one of the preceding claims, wherein a preliminary result is calculated locally from the recorded sensor data, which is sent to the analysis device (AE) to be validated or falsified in the analysis device (AE) on the basis of the received water sample.

**6.** RO system (RO) for producing ultrapure water with a sensor unit (S) for recording sensor data and with an electronic data interface (RO-S) which is used in a security system according to one of the preceding claims.

7. Server (SV) for the coordinated processing of security data from an RO system (RO) that is operated for at least one medical device (D), namely a dialysis device, wherein the server (SV) is used in a security system according to the preceding system claims, with:

- an electronic data interface (SV-S1) for receiving the sensor data recorded by the sensor unit (S);
- an analysis interface (SV-S2) for receiving the result data generated by the analysis device in electronic form; and comprising
- An evaluation device (P) for further processing the received data.

8. Server (SV) according to the immediately preceding claim, wherein the server (SV) further comprises a memory (MEM) for storing the received data and/or interacts with a database (DB).

9. Server (SV) according to one of the preceding claims directed to the server, wherein the server (SV) further comprises a control interface for controlling the RO system (RO) and/or the medical device(s) (D) based on the result data.

10. Method for security testing an RO system (RO) which is designed for use with at least one medical device (D), namely a dialysis device, in particular in a security system according to one of claims 1 to 5, comprising the following steps:

- Detecting (100) sensor data during operation of the RO system (RO) intended for the production of ultrapure water, wherein the sensor data recorded by a sensor unit comprise parameters that represent the correct functioning of the RO system (RO), namely two different parameters are detected before and after the membrane, namely the feed water conductivity and the permeate conductivity, as well as parameters for the retention capacity;
- Sending (200) the received sensor data to an external communication partner in electronic form;
- Receiving result data representing an analysis of a water sample from the RO system with regard to security requirements;
- wherein the sensor data and the result data are fed to a server (SV) for central processing and stored there.

11. Method according to the preceding method claim, in which the recording (100) of the sensor data takes place continuously or in a time-controlled manner during operation of the RO plant (RO) and/or after predefined events.

12. Method according to one of the preceding method claims, wherein the sensor data and the result data are fed to the server (SV) for central processing and, in particular, for statistical evaluation across RO plants.

13. Computer program product with a computer program with program sections for performing all method steps of the method according to one of the preceding method claims when the computer program is executed on a computer or an electronic device.

## Revendications

1. Système de sécurité électronique pour une installation RO (RO) conçue pour être utilisée avec au moins un appareil médical (D), à savoir un appareil de dialyse, comprenant :

- l’installation RO (RO), qui est destinée à la production d’eau ultra-pure et est conçue avec une unité de capteur (S) pour la détection de données de capteur, et l’installation RO (RO) comprenant une interface de données électronique (RO-S) pour envoyer les données de capteur détectées par l’unité de capteur (S), les données de capteur détectées par l’unité de capteur comprenant des paramètres qui représentent le fonctionnement correct de l’installation RO, à savoir que deux paramètres différents sont détectés avant et après la membrane, à savoir la conductivité de l’eau d’alimentation et la conductivité du perméat ainsi que des paramètres relatifs à la capacité de rétention ;
- un système de laboratoire comprenant au moins un dispositif de laboratoire et un dispositif d’analyse (AE), le au moins un dispositif de laboratoire étant destiné à analyser un échantillon d’eau de l’installation RO au regard des exigences de sécurité, et le système de laboratoire servant à détecter les contaminations de l’eau ainsi qu’à effectuer une analyse biologique et/ou chimique et des examens bactériologiques, et le dispositif d’analyse (AE) étant destiné à générer des données de résultat sur la base de l’analyse du dispositif technique de laboratoire, le dispositif d’analyse (AE) étant en outre équipé d’une interface d’analyse (AE-S) afin d’envoyer les données de résultat générées sous forme électronique ;

- un réseau (NW) pour l'échange de données entre les dispositifs médicaux du système de sécurité, à savoir entre l'installation RO (RO) et le dispositif d'analyse (AE),
- comprenant en outre un serveur avec un dispositif d'évaluation, sur lequel les données saisies et générées sont agrégées et qui sert au traitement concerté des données, les données de résultat étant calculées pour un ensemble d'appareils de dialyse, à savoir pour les appareils de dialyse alimentés par l'installation RO (RO) respective.

**2.** Système de sécurité selon la revendication 1, dans lequel l'installation RO (RO) et le système de laboratoire avec le dispositif d'analyse sont installés et mis à disposition sur deux systèmes d' s différents et séparés, l'installation RO et le dispositif d'analyse communiquant entre eux via une connexion de données qui peut être basée sur Internet.

**3.** Système de sécurité selon la revendication 1, dans lequel le serveur (SV) est destiné à recevoir les données des capteurs de l'installation RO (RO) et/ou les données de résultat du dispositif d'analyse (AE) et est en outre destiné à transmettre les données de résultat à l'installation RO (RO) et/ou à l'appareil médical (D) à des fins de commande et/ou de régulation.

**4.** Système de sécurité selon l'une des revendications précédentes, dans lequel le système comprend en outre une unité d'alimentation en eau (W) destinée à alimenter en eau l'installation RO (RO), l'unité d'alimentation en eau (W) comprenant une unité de mesure (M) pour mesurer les données de consommation d'eau, et l'unité de mesure (M) comprenant une interface de bus (MBUS) pour envoyer les données de consommation d'eau enregistrées.

**5.** Système de sécurité selon l'une des revendications précédentes, dans lequel un résultat préliminaire est calculé localement à partir des données de capteur enregistrées, lequel résultat est envoyé au dispositif d'analyse (AE) afin d'être validé ou invalidé dans le dispositif d'analyse (AE) sur la base de l'échantillon d'eau reçu.

**6.** Installation RO (RO) pour la production d'eau ultra-pure avec une unité de capteur (S) pour la saisie de données de capteur et avec une interface de données électronique (RO-S) qui est utilisée dans un système de sécurité selon l'une des revendications précédentes.

**7.** Serveur (SV) pour le traitement coordonné des données de sécurité d'une installation RO (RO) qui est exploitée pour au moins un appareil médical (D), à savoir un appareil de dialyse, le serveur (SV) étant utilisé dans un système de sécurité selon les revendications de système précédentes, avec :

- une interface de données électronique (SV-S1) pour recevoir les données de capteur enregistrées par l'unité de capteur (S) ;
- une interface d'analyse (SV-S2) pour recevoir les données de résultat générées par le dispositif d'analyse sous forme électronique ; et comprenant
- un dispositif d'évaluation (P) pour le traitement ultérieur des données reçues .

**8.** Serveur (SV) selon la revendication précédente, dans lequel le serveur (SV) comprend en outre une mémoire (MEM) pour stocker les données reçues et/ou interagit avec une base de données (DB).

**9.** Serveur (SV) selon l'une des revendications précédentes relatives au serveur, dans lequel le serveur (SV) comprend en outre une interface de commande pour commander l'installation RO (RO) et/ou le ou les appareils médicaux (D) sur la base des données de résultat.

**10.** Procédé de contrôle technique de sécurité d'une installation RO (RO) qui est conçue pour une application avec au moins un appareil médical (D), à savoir un appareil de dialyse, en particulier dans un système de sécurité selon l'une des revendications 1 à 5, comprenant les étapes suivantes :

- Enregistrement (100) des données du capteur pendant le fonctionnement de l'installation RO (RO) destinée à la production d'eau ultra-pure, les données de capteur enregistrées par une unité de capteur comprenant des paramètres qui représentent le bon fonctionnement de l'installation RO (RO), à savoir deux paramètres différents sont enregistrés avant et après la membrane, à savoir la conductivité de l'eau d'alimentation et la conductivité du perméat, ainsi que des paramètres relatifs à la capacité de rétention ;
- Envoi (200) des données de capteur enregistrées à un partenaire de communication externe sous forme électronique ;
- Réception de données de résultat représentant une analyse d'un échantillon d'eau de l'installation RO au regard des exigences de sécurité ;
- les données du capteur et les données de résultat étant transmises à un serveur (SV) pour un traitement centralisé et y étant enregistrées.

**11.** Procédé selon la revendication de procédé ci-dessus, dans lequel la saisie (100) des données du capteur s'effectue de manière continue ou programmée pendant le fonctionnement de l'installation RO (RO) et/ou après des événements prédéfinissables.

**12.** Procédé selon l'une des revendications de procédé précédentes, dans lequel les données du capteur et les données de résultat sont transmises au serveur (SV) pour un traitement centralisé et en particulier une évaluation statistique couvrant plusieurs installations RO.

**13.** Produit logiciel comprenant un programme informatique avec des sections de programme pour exécuter toutes les étapes du procédé selon l'une des revendications de procédé précédentes, lorsque le programme informatique est exécuté sur un ordinateur ou un appareil électronique.

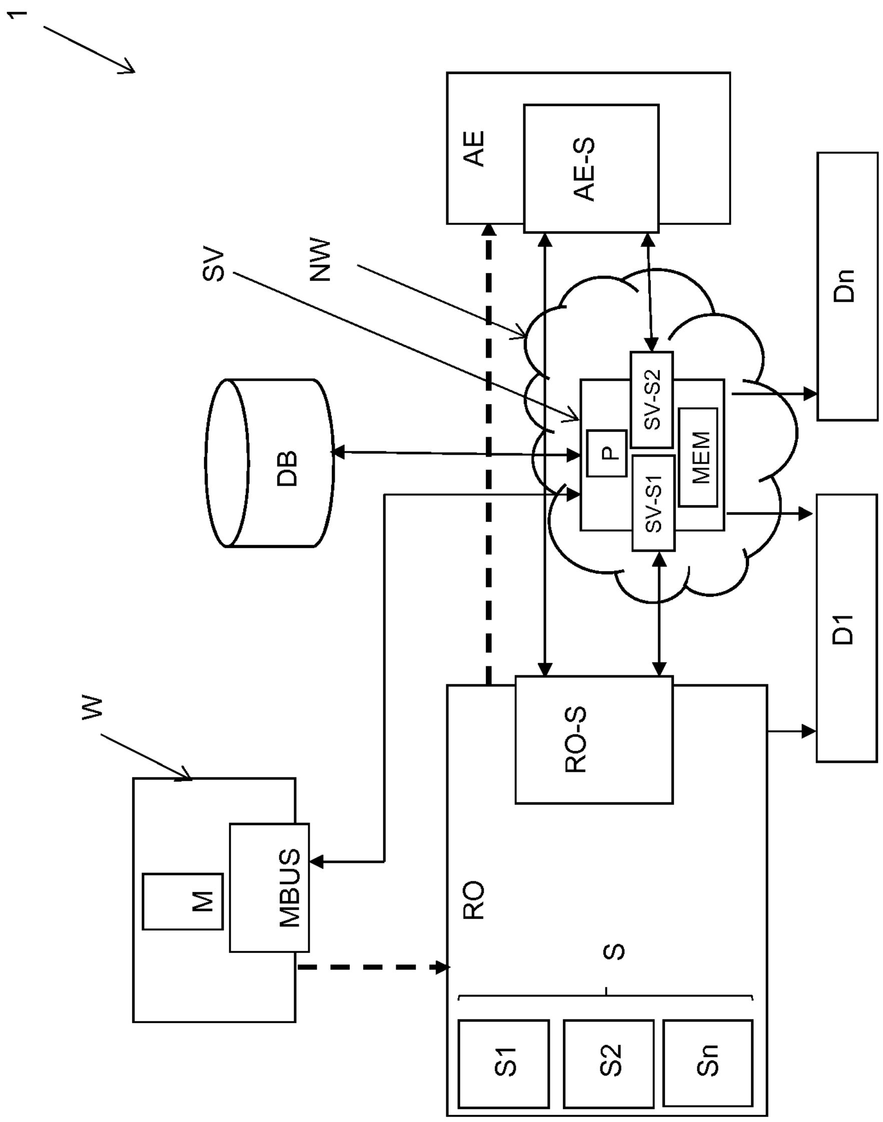


FIG. 1

FIG. 2

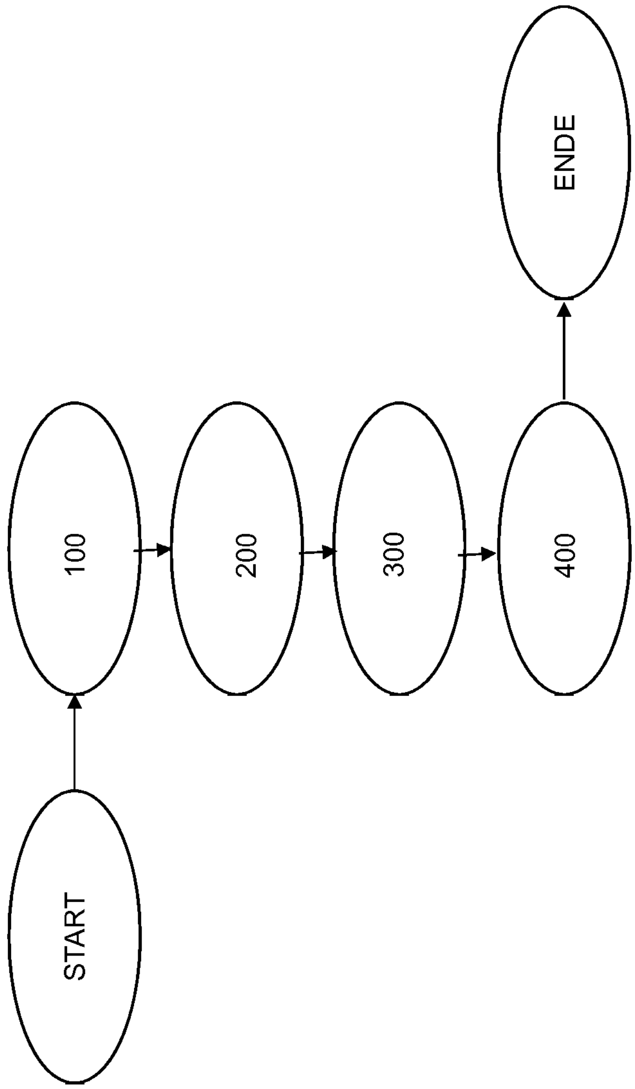

FIG. 3

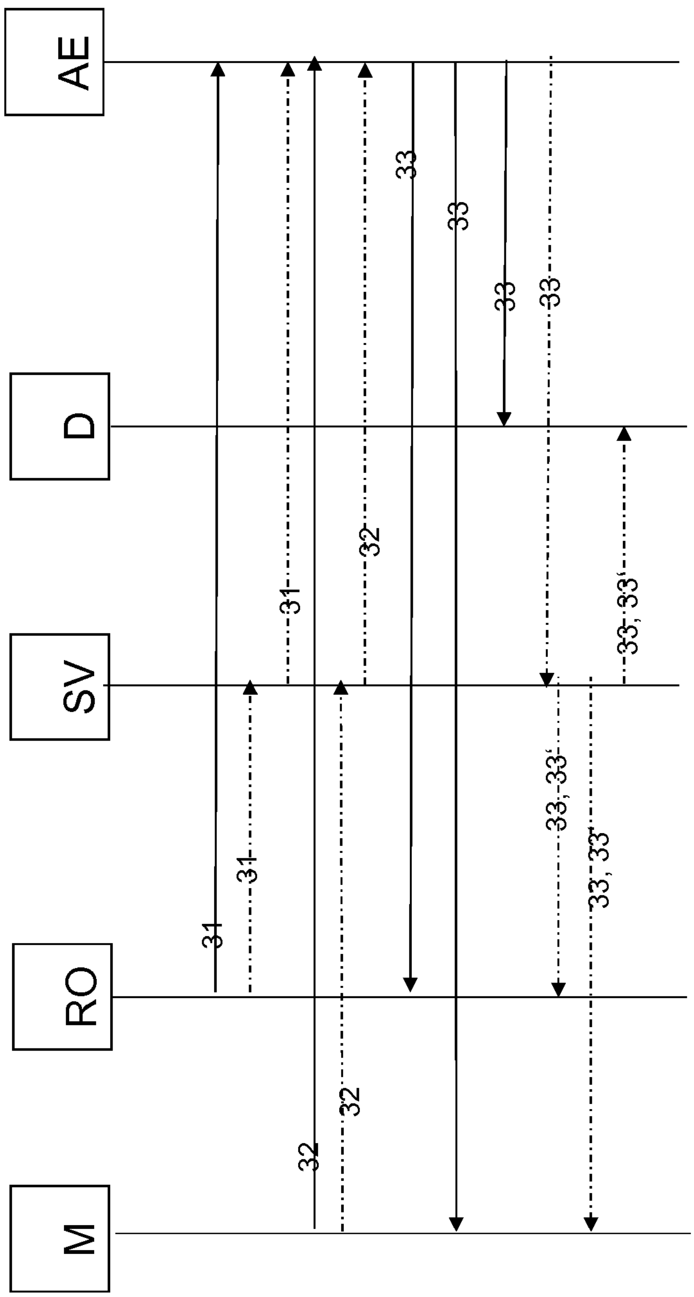

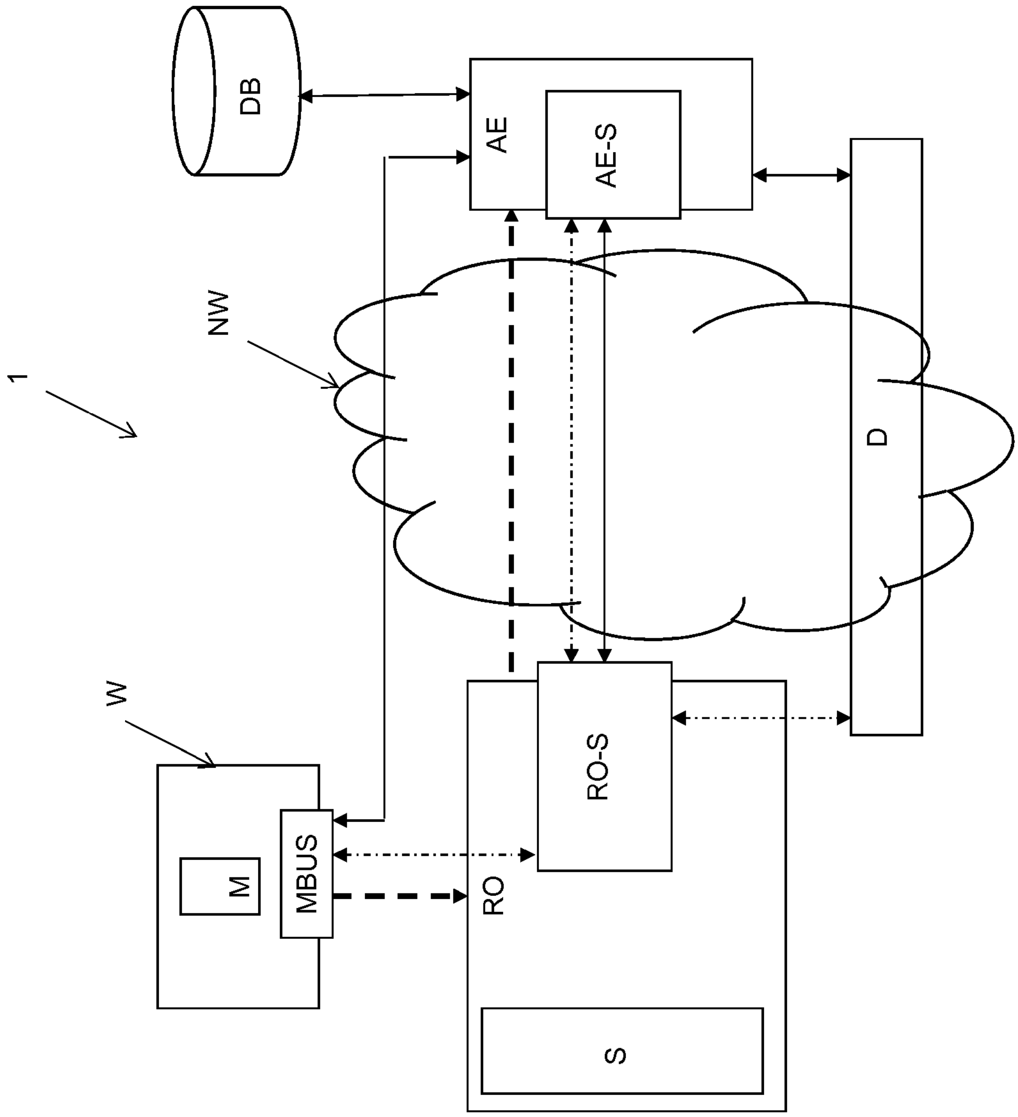


FIG. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010024963 A1
- US 20110284480 A1
- CN 106119889 A
- US 20020077777 A1
- US 20040138840 A1
- FR 2911687
- US 20020130069 A1
- WO 0036412 A